# EUROPEAN PATENT APPLICATION

(11) **EP 4 821 931 A1**
(43) Date of publication of application: **14.10.2026**
(21) Application number: 24901085.1
(22) Date of filing: 05.12.2024
(51) Int. Cl.: C07D 487/04, A61K 31/519

(54) **METHOD FOR PREPARATION OF 1-((3S,4R)-3-((2-((1-ETHYL-1H-PYRAZOL-4-YL)AMINO)-7H-PYRROLO[2,3-D]PYRIMIDIN-4-YL)OXY)-4-FLUOROPIPERIDIN-1-YL)PROP-2-EN-1-ONE, AND INTERMEDIATE COMPOUNDS THEREOF**

(30) Priority: 05.12.2023 KR 20230174736; 16.01.2024 KR 20240006933
(71) Applicant: Daewoong Pharmaceutical Co., Ltd., Hwaseong-si, Gyenoggi-do 18623 (KR)
(72) Inventor: PARK, Joon Seok, Gyeonggi-do 17000 (KR); HYUN, Hyae Jung, Seongnam-si, Gyeonggi-do 13568 (KR); LEE, Joon-Hwan, Suwon-si, Gyeonggi-do 16693 (KR); JUN, Sun Ah, Yongin-si, Gyeonggi-do 17027 (KR); KIM, Namyoun, Yongin-si, Gyeonggi-do 17002 (KR); KIM, Wonsun, Hwaseong-si, Gyeonggi-do 18430 (KR); KIM, Cheolwoong, Suwon-si, Gyeonggi-do 16684 (KR)
(74) Representative: Germain Maureau
(86) International application number: PCT/KR2024/019811
(87) International publication number: WO 2025/121899

(57) **Abstract**

The present disclosure relates to a method for preparation of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one, and intermediate compounds thereof.

## Description

### [TECHNICAL FIELD]

### CROSS-REFERENCE TO RELATED APPLICATION(S)

This application claims priority to and the benefit of Korean Patent Application No. 10-2023-0174736, filed on December 5, 2023, and Korean Patent Application No. 10-2024-0006933, filed on January 16, 2024, with the Korean Intellectual Property Office, the disclosures of which are incorporated herein by reference in their entirety.

The present disclosure relates to a method for preparation of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one, and intermediate compounds thereof.

### [BACKGROUND]

Protein kinase is an enzyme that catalyzes phosphorylation of specific residues of other proteins, and plays an important role in signal-transduction pathways that transduce extracellular signals to the nucleus. Further, it is involved in various diseases in vivo. In the onset or development of inflammatory disease, autoimmune disease, proliferative disease or hyperproliferative disease, and/or immunity mediated disease, there is various evidence that T-cells (or T-lymphocytes) and B-cells (or B-lymphocytes) play an important role.

Janus kinase (hereinafter referred to as "JAK") is a cytoplasmic protein tyrosine kinase that plays pivotal roles in regulating cell function in the lymphohematopoietic system. Cytokines are known to play an important role in regulating inflammation, immunity and normal cell function, and JAK activates STAT (Signal Transducer and Activators of Transcription) proteins through tyrosine phosphorylation to provide rapid signaling pathways to cytokines. JAK/STAT signaling is known to be associated with allergies, asthma, autoimmune diseases (e.g., transplant rejection, rheumatoid arthritis, amyotrophic lateral sclerosis, multiple sclerosis etc.), solid cancers, blood cancers (e.g., leukemia, lymphoma and so on).

The JAK family is classified into four members: JAK1, JAK2, JAK3, and TYK2. Members of the JAK family pair with each other to mediate signals from a variety of cytokines. It includes JAK2 and JAK1 associated with hematopoietic growth factor signaling, and a combination of TYK2 and AK2 is important for interferon signaling and contributes to host tolerance. JAK2 can induce anemia, thrombocytopenia, leukopenia, especially when it is involved in the hematopoietic growth factor signaling and causes excessive inhibition.

The expression of JAK1, JAK2, and TYK2 was found to be widely distributed, whereas the expression of JAK3 was restricted to lymphocytes and is associated with signaling for the common gamma chains, members of IL-2, IL-4, IL-7, IL-9, IL-15 and IL-21 receptors, particularly the common gamma chain of the IL-2 family. As soon as the cytokine is bound, the receptor carries adjacent JAK3 nearby, which induces autophosphorylation of the β-chain C-terminus. As a result, it causes activation of the STAT protein, which is an important step in retransmitting the signal to the nucleus. JAK3 controls the signal pathways of various cytokines through this process. This makes JAK3 as an attractive target for immunosuppression.

B cells play an important role in the development of autoimmune and/or inflammatory diseases. Protein-based therapeutic agents that reduce B cells, for example Rituxan, are effective in autoantibody-induced inflammatory diseases such as rheumatoid arthritis. Thus, protein kinase inhibitors that play a role in B cell activation are useful therapeutic agents for the treatment of B cellmediated diseases, for example, for the production of autoantibodies.

Signal transduction through B cell receptor(BCR) regulates various B cell responses, including proliferation and differentiation into mature antibodyproducing cells. BCR is an important regulatory element of B cell activity, and abnormal signal transduction can cause the formation of pathogenic autoantibodies leading to a plurality of autoimmune and/or inflammatory diseases and the proliferation of deregulated B cell.

Bruton's tyrosine kinase (hereinafter, referred to as "BTK") is an important regulator of the development, activation, signaling and survival of B-cells. BTK is involved in signal transduction pathways initiated by binding various extracellular ligands to their cell surface receptors. Following ligation of the B cell antigen receptor (BCR), the activity of BTK by the coincident action of the protein tyrosine kinases Lyn and Syk is required for the induction of the phospholipase C-γ2-mediated calcium mobilization. Therefore, inhibition of BTK can be a useful therapeutic approach in blocking the onset process of B-cell mediated diseases.

As mentioned above, Janus kinase and TEC-based kinases plays an important role in the activation of T-cells and/or B-cells involved in the development of inflammatory diseases, autoimmune diseases, proliferative diseases or hyperproliferative diseases, and immunity mediated diseases. Therefore, the development of substances that effectively inhibit these diseases can be useful as a related therapeutic agent. Specific examples of the diseases which can be treated and prevented include cancer, transplant rejection, multiple sclerosis, rheumatoid arthritis, psoriatic arthritis, psoriasis, asthma, allergic dermatitis, atopic dermatitis, eczema, type I diabetes, diabetic complication, ulcerative colitis, Crohn's disease, autoimmune thyroid disorder, systemic depilation, Sjogren's syndrome and the like.

JAK3 kinase inhibitor, tofacitinib (CP-690550) (Pfizer Inc.) is currently approved and marketed for the treatment of rheumatoid arthritis. In addition, a BTK kinase inhibitor, ibrutinib (PCI-32765) (Pharmacyclics) is in a clinical stage, but severe side effects such as skin rash and diarrhea have been reported in clinical cases. Thus, there is a need to develop a more stable and effective substance that inhibits JAK and/or BTK (see, Nat Rev Rheumatol. 2009 Jun 5(6) 317-24; Expert Opin Investig Drugs. 2014 Aug 23(8) 1067-77; Drug Discov Today 2014 Aug 19(8) 1200-4; WO2002/096909; WO2010-009342).

Therefore, the present inventors has found that an oxy-fluoropiperidine derivative, which has a chemical structure different from that of kinase inhibitors reported to date, has excellent inhibitory activity as a kinase inhibitor. In this regard, the present inventors have conducted intensive research on a preparation method capable of preparing a new oxy-fluoropiperidine derivative, and as a result, found that the preparation method described below allows for commercial mass production, and furthermore, reduces impurities while improving the yield and quality as a whole, and completed the present disclosure.

### [DETAILED DESCRIPTION OF THE INVENTION]

### [Technical Problem]

It is an object of the present disclosure to provide a method for preparing 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one.

It is another object of the present disclosure to provide a method for preparing a tartrate salt of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one.

It is another object of the present disclosure to provide a novel intermediate compound obtained in the process of preparing 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one.

### [Technical Solution]

In order to achieve the above objects, there is provided a method for preparing a tartrate salt of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one, which is a compound represented by the following Chemical Formula 1. More specifically, the preparation method comprises the following steps, wherein at least one of the following steps 1 to 4 further comprises a crystallization step:
(step 1) reacting a compound represented by the following Chemical Formula 1-1 and a compound represented by the following Chemical Formula 1-2 in the presence of a base to prepare a compound represented by the following Chemical Formula 1-3;
(step 2) reacting a compound represented by the following Chemical Formula 1-3 and a compound represented by the following Chemical Formula 1-4 in the presence of a palladium catalyst and a base to prepare a compound represented by the following Chemical Formula 1-5;
(step 3) reacting a compound represented by the following Chemical Formula 1-5 with an acid to prepare a compound represented by the following Chemical Formula 1-6; and
(step 4) reacting a compound represented by the following Chemical Formula 1-6 and a compound represented by the following Chemical Formula 1-7 in the presence of a base to prepare a compound represented by the following Chemical Formula 1: 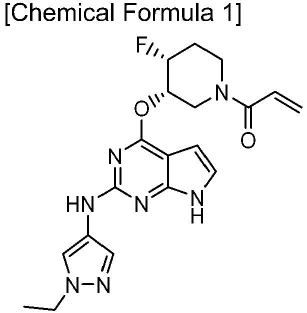 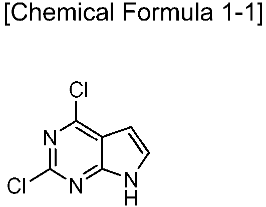 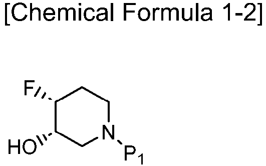 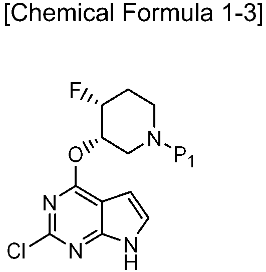 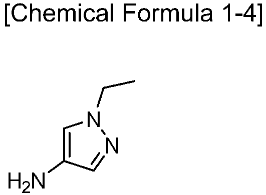 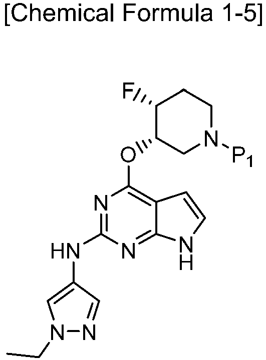 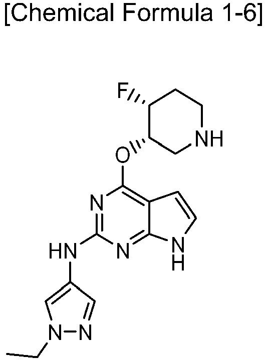 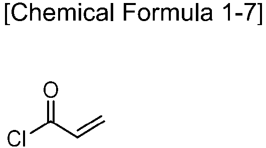
wherein in Chemical Formulas 1-1 to 1-7,
P₁ means a protecting group.

Further provided herein is a method for preparing a compound represented by the following Chemical Formula 1,
comprising a compound represented by the following Chemical Formula 1-5 as an intermediate: 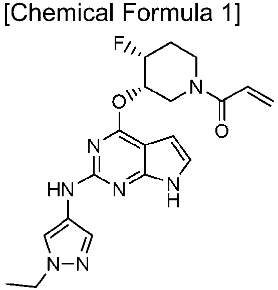 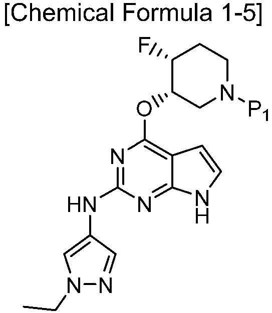
wherein in Chemical Formula 1-5,
P₁ means a protecting group.

Further, there is provided a compound represented by the following Chemical Formula 1-5: 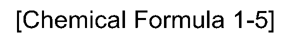 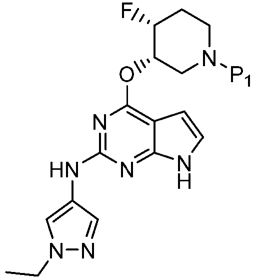 wherein in Chemical Formula 1-5,
P₁ means a protecting group.

The preparation method does not include a separate substitution reaction of a protective group, and therefore the process steps are shortened compared with a conventional preparation method, and also does not go through a purification step by a column, and therefore is improved in yield and/or purity of the final product. Further, the preparation method involves crystallization purification in at least one steps, which is advantageous for mass production. In addition, the preparation method does not use the explosive strong bases (NaH, etc.) that were previously used, thereby ensuring safety during the process, and the reaction temperatures in some steps are lower than before, thereby being advantageous for application to general production plants and having economic effects.

The compound represented by Chemical Formula 1 is prepared through the following Reaction Scheme 1: 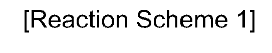 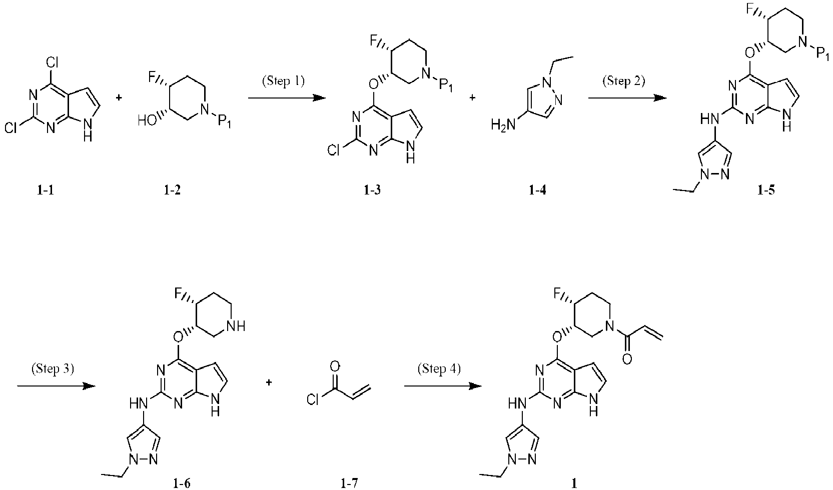 in Reaction Scheme 1, P₁ means a protecting group.

More specifically, P₁ may be any one protecting group selected from the group consisting of tertbutoxycarbonyl(Boc), carbobenzyloxy(Cbz), paramethoxybenzylcarbonyl(Moz), 9-fluorenylmethyloxycarbonyl(Fmoc), acetyl(Ac), benzoyl(Bz), benzyl(Bn), and para-methoxybenzyl(PMB).

Meanwhile, each step described below may further comprise a purification step after the reaction in order to prepare a compound with high purity and yield. In one example of the purification method, extraction using a difference in solubility, adsorption using activated carbon and/or silica-based materials, filtration, precipitation, crystallization, etc. may be used. However, the purification method does not use column chromatography, which is not suitable for mass production. Preferably, the purification step may be carried out as described in each step.

In addition, each step may further comprise a crystallization step using a crystallization solvent as the final step. The crystallization step may be carried out after the purification step if the purification step is carried out, and it may be carried out after the reaction if the purification step is not carried out. By further including the crystallization step, mass production is made possible. The crystallization solvent may be used according to the product of each step, but is not particularly limited. Preferably, the crystallization step may be carried out as described in each step.

### (Step 1)

The step 1 is a step of reacting the compound represented by Chemical Formula 1-1 and the compound represented by Chemical Formula 1-2 in the presence of a base to prepare a compound represented by Chemical Formula 1-3. The reaction is a nucleophilic aromatic substitution reaction, which is carried out in the presence of a base to make the added compound represented by Chemical Formula 1-2 into a nucleophile.

In the step 1, the compound represented by Chemical Formula 1-2 can be used in an amount of 0.1 to 10 equivalents based on 1 equivalent of the compound represented by Chemical Formula 1-1. Specifically, the compound represented by Chemical Formula 1-2 can be used in an amount of 0.2 to 5 equivalents, 0.3 to 3 equivalents, 0.5 to 2 equivalents, 0.7 to 1.5 equivalents, 1 to 1.5 equivalents, 1 to 1.3 equivalents, 1 to 1.05 equivalents, or 1.05 equivalents, based on 1 equivalent of the compound represented by Chemical Formula 1-1.

Further, in the step 1, the base may be at least one selected from the group consisting of potassium tert-butoxide, sodium tert-butoxide, potassium methoxide, sodium methoxide, potassium tert-pentoxide, sodium tert-pentoxide, triethylamine, diisopropylethylamine, sodium hydroxide, potassium hydroxide, lithium hydroxide, potassium carbonate, calcium carbonate, sodium carbonate, potassium hydrogen carbonate, sodium hydrogen carbonate and cesium carbonate. Among these bases, potassium tert-butoxide is preferably used in terms of reaction speed and yield. The base used is sufficient only by a non-explosive base, safety is secured, which is advantageous for industrialization.

And, the base can be used in an amount of 0.1 to 10 equivalents based on 1 equivalent of the compound represented by Chemical Formula 1-1. Preferably, the base can be used in an amount of 0.5 to 5 equivalents, 1 to 5 equivalents, 2 to 5 equivalents, 2 to 3 equivalents, or 3 equivalents, based on 1 equivalent of the compound represented by Chemical Formula 1-1. If the base is used in an excessively small amount, there is a risk that the reaction time is prolonged, and even if the base is used in an excessively large amount, there is no difference in the reaction time, and so it is preferable to use the base in the above-mentioned range.

In the step 1, the reaction may be carried out in one or more organic solvents selected from the group consisting of tetrahydrofuran(THF), 1,4-dioxane, methanol, ethanol, isopropanol, 1-butanol, 2-butanol, acetone, methyl ethyl ketone, ethyl acetate, methyl tert-butyl ether, acetonitrile, toluene, heptane, 3-methyltetrahydrofuran, dichloromethane, methyl isobutyl ketone, dimethyl formamide(DMF), dimethyl acetamide(DMAC), and dimethyl sulfoxide(DMSO). As an example, one type of the solvent may be used, or a mixed solvent of two or more types may be used. Preferably, 1,4-dioxane may be used as the organic solvent. 1,4-Dioxane has the advantage that it can produce products of higher purity than other solvents and it is easy to separate layers during extraction. Other solvents have the disadvantage that they produce relatively large amounts of impurities or have slow reaction rates.

Further, the organic solvent may be used in an amount of 5 to 30 times (mL/g), more specifically, 5 to 15 times (mL/g), based on the weight of the compound represented by Chemical Formula 1-1.

Further, the reaction can be carried out at 30 to 60 °C for 2 to 10 hours. If the reaction is carried out under temperature conditions lower than the above-mentioned range and/or for a short reaction time, the reaction may not proceed sufficiently, which may lead to the deterioration of a production yield. In addition, even if the reaction is carried out under temperature conditions higher than the above-mentioned range and/or for a long reaction time, the production yield is not substantially increased, which is not desirable in terms of process cost.

Meanwhile, after the reaction of the step 1 is completed, the method may further comprise a purification step of extracting the reaction product using ethyl acetate and/or water. Specifically, the reaction product may be extracted using ethyl acetate and water. That is, the compound represented by Chemical Formula 1-3 may be extracted using ethyl acetate and water after the completion of the reaction. The extraction using ethyl acetate and water may be carried out by a method in which the mixture containing the reaction product and each solvent is stirred for 30 minutes or more, allowed to stand for 30 minutes or more and then separated into layers, so that an aqueous layer (lower layer) is discarded and an organic layer (upper layer) is taken. The extraction using ethyl acetate and water may be repeated one or more times. In the case of such an extraction, impurities having a relative retention time of less than 1.0 as separated by high performance liquid chromatography(HPLC) can be effectively removed by the aqueous layer, compared with the case of extraction using a dichloromethane/water mixed solvent.

In addition, the step 1 may further comprise a step of filtering the extracted mixture, followed by vacuum concentration and/or azeotropic concentration. This vacuum concentration and/or azeotropic concentration step is preferably carried out after the extraction step with ethyl acetate and/or water.

In addition, the step 1 may further include a step of crystallizing a reaction product in order to purify the compound represented by Chemical Formula 1-3. Such a crystallization step is preferably carried out after the extraction and purification steps. In other words, the step 1 can be carried out by a step in which, after the extraction and purification steps, a crystallization solvent is added to the concentrated product and stirred at a temperature of about 20 to 50 °C for 30 minutes to 4 hours to prepare the compound represented by Chemical Formula 1-3.

When a reaction product is prepared by crystallization using a crystallization in this way, unlike column chromatography in which purification is carried out using a silica column, it is possible to apply to industrialization, which is advantageous in terms of industrial mass production, and has the advantage of being able to obtain a high yield.

Specifically, the crystallization step can be carried out by crystallization using water as a crystallization solvent. The crystallization step can be carried out by repeating the crystallization process one or more times, which proceeds in the order of 'adding the crystallization solvent and then stirring'. In this manner, when the crystallization process is repeated while adding the solvent multiple times instead of adding the solvent at once, it is possible to obtain even the crystals that adhere to the wall surface during the solvent addition process, thereby preventing a decrease in yield, effectively removing by-products remaining after the reaction, and crystallizing an uncrystallized product through repeated crystallization processes. Thereby, compared with a process of purifying in a slurry form using a solvent such as ethyl acetate, a high-purity compound can be obtained in a high yield.

The crystallization solvent used in one crystallization process may be used in an amount of volume(mL/g) of 3 to 20 times, more specifically, in an amount of volume(mL/g) of 3 to 10 times, relative to the weight of the compound represented by Chemical Formula 1-1.

One crystallization process may be carried out by stirring at a temperature range of 5 to 30 °C, preferably at room temperature (20 to 25 °C) for 10 minutes to 2 hours. The process time can be appropriately adjusted, for example, by increasing it when the number of repetitions of the crystallization process is small, and decreasing it when the number of repetitions is large.

That is, when all the steps of purifying and crystallizing a reaction product of the step 1 are carried out to prepare the compound represented by Chemical Formula 1-3, the step 1 may include the following steps, but is not limited to these steps:
a step of reacting the compound represented by Chemical Formula 1-1 with the compound represented by Chemical Formula 1-2 in the presence of a base;
a step of adding ethyl acetate and water to discard the aqueous layer (lower layer) and separate and extract the organic layer (upper layer), and step of vacuum concentrating and/or azeotropically concentrating the purified mixture; and
a step of crystallizing the concentrate.

After purifying the compound represented by Chemical Formula 1-3, the compound may be dried at 40 to 60 °C for 10 to 14 hours to reduce the moisture content contained in the compound represented by Chemical Formula 1-3. More specifically, if the moisture content of the compound represented by Chemical Formula 1-3 is significantly reduced by drying at 50 to 60 °C, the conversion rate of the next step (i.e., step 2 below) can be increased.

### (Step 2 and Step 3)

The step 2 is a step of reacting the compound represented by Chemical Formula 1-3 and the compound represented by Chemical Formula 1-4 in the presence of a palladium catalyst and a base to prepare the compound represented by Chemical Formula 1-5, wherein a Buchwald-Hartwig amination reaction is carried out. The Buchwald-Hartwig amination reaction is a reaction in which a carbon and nitrogen bond is formed through a palladium catalyzed coupling reaction of an amine and an aryl halide. In addition, the step 3 is a step of reacting the compound represented by Chemical Formula 1-5 with an acid to prepare the compound represented by Chemical Formula 1-6, which is a step for removing the protecting group P₁ contained in the Chemical Formula 1-5.

The concentrated residual liquid containing the compound represented by Chemical Formula 1-5 produced in the step 2 can be used as it is in the next step without a separate purification process to ensure a stable reaction without a decrease in yield or purity, etc. Thus, in consideration of the economic effect caused by omitting the purification step and the ease and convenience of the process, the concentrated residual liquid of the step 2 can be used as it is to configure the step 2 and step 3 in-situ.

In the step 2, the compound represented by Chemical Formula 1-4 can be used in an amount of 0.1 to 10 equivalents based on 1 equivalent of the compound represented by Chemical Formula 1-3. Specifically, the compound represented by Chemical Formula 1-4 can be used in an amount of 0.2 to 5 equivalents, 0.5 to 5 equivalents, 0.5 to 3 equivalents, 0.5 to 2 equivalents, 0.5 to 1.5 equivalents, 1 to 1.5 equivalents, or 1.5 equivalents, based on 1 equivalent of the compound represented by Chemical Formula 1-3.

In the step 2, the palladium catalyst may be a palladium(0) catalyst, where the valence of palladium(Pd) in the compound is zero, or a palladium(II) catalyst, where the valence is +2. For example, the palladium catalyst may be at least one selected from the group consisting of tris(dibenzylideneacetone)dipalladium(0), tetrakis(triphenylphosphine)palladium(0), bis[tris(2-methylphenyl)phosphine]palladium, palladium(II)acetate, 1,1-(bis(diphenylphosphino)ferrocene)palladium(II)dichloride, and palladium(II)chloride.

Further, in the step 2, the base may be at least one selected from the group consisting of potassium tert-butoxide, sodium tert-butoxide, potassium methoxide, sodium methoxide, potassium tert-pentoxide, sodium tert-pentoxide, triethylamine, diisopropylethylamine, sodium hydroxide, potassium hydroxide, lithium hydroxide, potassium carbonate, calcium carbonate, sodium carbonate, potassium hydrogen carbonate, sodium hydrogen carbonate and cesium carbonate. The base used is sufficient only by a non-explosive base, safety is secured, which is advantageous for industrialization.

Further, the reaction may be carried out in the presence of a phosphine-based compound together with the palladium catalyst and the base. As the phosphine-based compound, at least one selected from the group consisting of 2,2'-bis(diphenylphosphino)-1,1'-binaphthyl, 4,5-bis(diphenylphosphino)-9,9-dimethylxanthene, 2-(dicyclohexylphosphino)-3,6-dimethoxy-2,4'6'-triisopropyl-1,1'-biphenyl, 2-di-tert-butylphosphino-2',4',6'-triisopropylbiphenyl and dicyclohexylphosphino-2',6'-diisopropoxybiphenyl may be used.

Preferably, in the step 2, the base may be used in an amount of 0.1 to 10 equivalents based on 1 equivalent of the compound represented by Chemical Formula 1-3. Preferably, the base may be used in an amount of 0.5 to 5 equivalents, 1 to 5 equivalents, 2 to 5 equivalents, 3 to 5 equivalents, or 3 equivalents, based on 1 equivalent of the compound represented by Chemical Formula 1-3. If the base is used in an excessively small amount, there is a risk that the reaction time is prolonged, and even if the base is used in an excessively large amount, there is no difference in the reaction time, and thus the base is used preferably in the above-mentioned range.

As the reaction solvent in the step 2, a solvent that is inactive to the amine substitution reaction can be used. For example, the reaction may be carried out in one or more organic solvents selected from the group consisting of tetrahydrofuran(THF), 1,4-dioxane, sec-butyl alcohol, methanol, ethanol, isopropanol, 1-butanol, 2-butanol, tert-butyl alcohol, acetone, methyl ethyl ketone, ethyl acetate, methyl tert-butyl ether, acetonitrile, toluene, xylene, heptane, 3-methyltetrahydrofuran, dichloromethane, methyl isobutyl ketone, dimethyl formamide(DMF), dimethyl acetamide(DMAC), and dimethyl sulfoxide(DMSO). As an example, one type of the solvent may be used, or a mixed solvent of two or more types may be used, but sec-butyl alcohol is preferable in terms of reaction speed and yield.

The organic solvent may be used in an amount of volume(mL/g) of 0.5 to 10 times, specifically, in an amount of volume(mL/g) of 4 to 8 times, relative to the weight of the compound represented by Chemical Formula 1-3. If the solvent is excessive during the reaction of the step 2, there is a problem that the reaction does not proceed.

Further, the reaction may be carried out at a temperature of 80 to 100 °C for 1 to 10 hours. If the reaction is carried out under temperature conditions lower than the above-mentioned range and/or for a short reaction time, the reaction may not proceed sufficiently, which may lead to the deterioration of a production yield. In addition, even if the reaction is carried out under temperature conditions higher than the above-mentioned range and/or for a long reaction time, the production yield is not substantially increased, which is not preferable in terms of process cost. More specifically, the reaction can be carried out at a temperature of 85 to 95 °C or 88 to 92 °C for 1 to 6 hours, 1 to 4 hours, preferably 1 to 3 hours, and more preferably 1.5 to 2 hours. The reaction temperature is in a range far below the boiling point of the reaction solvent, which is significantly lower than conventional reaction temperatures, thereby making it easy to apply in the process and reducing time and costs.

Further, the reaction can be carried out in a commonly known reactor when using the combination of the above-mentioned palladium catalyst, base, and phosphine-based compound. Thereby, the step 2 can be applied as one step in the process for mass-producing the compound represented by Chemical Formula 1.

Meanwhile, after the reaction of the step 2 is completed, the method may further comprise a purification step of extracting the reaction product using ethyl acetate and water or using ethyl acetate and a sodium chloride aqueous solution. Specifically, the reaction product may be extracted by using ethyl acetate and water or using ethyl acetate and a sodium chloride aqueous solution or using both of the above methods. That is, the compound represented by Chemical Formula 1-5 may be extracted by using ethyl acetate and water or using ethyl acetate and a sodium chloride aqueous solution after the reaction is completed. The extraction using ethyl acetate and water or using ethyl acetate and a sodium chloride aqueous solution may be carried out by a method in which the mixture containing the reaction product and each solvent is stirred for 30 minutes or more, allowed stand for 30 minutes or more and then separated into layers, so that an aqueous layer (lower layer) is discarded and an organic layer (upper layer) is taken. The extraction using ethyl acetate and water may be repeated one or more times. In the case of such an extraction, impurities having a relative retention time of less than 1.0 as separated by high performance liquid chromatography(HPLC) can be effectively removed by the aqueous layer, compared with the case of extraction using a dichloromethane/water mixed solvent.

In addition, the step 2 may further comprise a step of vacuum concentrating and/or azeotropically concentrating the extracted mixture. The vacuum concentration and/or azeotropically concentrating steps are preferably carried out after the extraction step using the ethyl acetate and water; or the ethyl acetate and sodium chloride aqueous solution.

That is, when all the steps of purifying the reaction product of the step 2 are carried out to prepare the compound represented by Chemical Formula 1-5, the step 2 may include the following steps, but is not limited to these steps:
a step of reacting the compound represented by Chemical Formula 1-3 and the compound represented by Chemical Formula 1-4 in the presence of a palladium catalyst and a base;
a step of adding ethyl acetate and water, or ethyl acetate and an aqueous sodium chloride solution to discard the aqueous layer (lower layer) and separate and extract the organic layer (upper layer); and
a step of vacuum concentrating and/or azeotropically concentrating the purified mixture.

Meanwhile, after the reaction is completed, the process of step 2 and step 3 described below can be carried out in-situ in the same reaction vessel, without a process of purifying or separating the compound represented by Chemical Formula 1-5. In this manner, since the process of purifying and separating the compound in the step 2 is not required, the method for preparing the compound represented by Chemical Formula 1 including the step 2 may be advantageous in terms of industrial mass production of the compound.

The step 3 is a step of reacting the compound represented by Chemical Formula 1-5 with an acid to prepare the compound represented by Chemical Formula 1-6, which is a step of removing the protecting group P₁ contained in Chemical Formula 1-5.

As the acid used in the reaction of the step 3, at least one selected from the group consisting of hydrochloric acid, acetic acid, trichloroacetic acid, trifluoroacetic acid, bromic acid, sulfuric acid, nitric acid, and iodic acid may be used. Among these acids, it is preferable to use hydrochloric acid when considering the yield and cost. Here, concentrated hydrochloric acid(c-HCl) may be used as the hydrochloric acid. For example, concentrated hydrochloric acid with a concentration of about 30 to 40 wt.% can be used.

The acid can be used in an amount of volume (mL/g) of 1 to 10 times, more specifically, in an amount of volume (mL/g) of 2 to 5 times, relative to the weight of the compound represented by Chemical Formula 1-5.

The reaction solvent of the step 3 may be one or more organic solvents selected from the group consisting of ethyl acetate, dichloromethane, butyl acetate, trichloromethane, methanol, ethanol, isopropanol, 1-butanol, 2-butanol, acetone, methyl ethyl ketone, methyl tert-butyl ether, acetonitrile, toluene, heptane, tetrahydrofuran, 3-methyltetrahydrofuran, 1,4-dioxane, methyl isobutyl ketone, dimethylformamide(DMF), dimethylacetamide(DMAC), and dimethyl sulfoxide(DMSO). As an example, one type of the solvent may be used, or a mixed solvent of two or more types may be used.

The organic solvent may be used in an amount of volume (mL/g) of 5 to 30 times, more specifically, in an amount of volume (mL/g) of 5 to 15 times, or 5 to 6 times, relative to the weight of the compound represented by Chemical Formula 1-5.

Further, the reaction can be carried out at 0°C to 50°C for 1 hour to 10 hours, preferably 3 hours to 5 hours. The removal reaction of the tert-butoxycarbonyl group, which is a protecting group, corresponds to a reaction that easily occurs without additional heat treatment, and can proceed within the above temperature range. However, if the reaction time is less than 1 hour, the reaction may not proceed sufficiently, and if the reaction time exceeds 10 hours, the production yield is not substantially increased, and thus the above-mentioned reaction time is preferred.

Meanwhile, after the reaction of the step 3 is completed, the method may further comprise a purification step of extracting the reaction product. Preferably, the solvent used for the extraction is not limited, but as an example, the extraction step may extract the reaction product using dichloromethane and water or using ethyl acetate and water. That is, the compound represented by Chemical Formula 1-6 may be extracted using dichloromethane and water or using ethyl acetate and water or both of the above methods after the reaction is completed.

The extraction using dichloromethane and water can be carried out by a method in which the mixture containing the reaction product and each solvent is stirred for 30 minutes or more, allowed to stand for 30 minutes or more and then separated into layers, so that an organic layer (lower layer) is discarded, and an aqueous layer (upper layer) is taken. The extraction using ethyl acetate and water can be carried out by a method in which the mixture containing the reaction product and each solvent is stirred for 30 minutes or more, allowed to stand for 30 minutes or more and then separated into layers, so that an aqueous layer (lower layer) is discarded, and an organic layer (upper layer) is taken. The extraction using dichloromethane and water or the extraction using ethyl acetate and water may be repeated one or more times. Meanwhile, the extraction using ethyl acetate and water is preferably carried out in the presence of a base such as sodium hydroxide while maintaining the pH of the reaction product at 9 to 11.

In addition, the step 3 may further comprise a step of vacuum concentrating and/or azeotropically concentrating the extracted mixture. This vacuum concentration and/or azeotropic concentration steps are preferably carried out after the extraction step using dichloromethane and water; or ethyl acetate and water.

And, the step 3 may further comprise a step of crystallizing a reaction product in order to purify the compound represented by Chemical Formula 1-6. This crystallization step is preferably carried out after the extraction purification step. In other words, the step 3 may be carried out by a step of adding a crystallization solvent to the concentrated product after the extraction purification step and stirring the mixture at a temperature of about 20 to 40 °C for 30 minutes to 4 hours to prepare the compound represented by Chemical Formula 1-6. Preferably, the crystallization step may be carried out at room temperature of 20 to 30 °C, or 20 to 25 °C.

In this way, when a reaction product is prepared through crystallization using a crystallization solvent, unlike the case of purification using a silica column, it is possible to apply to industrialization, which is advantageous in terms of industrial mass production, and has the advantage of being able to obtain a high yield.

Specifically, the crystallization step can be carried out by crystallization using methyl isobutyl ketone or normal-heptane as a crystallization solvent. When both of the above solvents are used, the order is not limited, but it is preferable to proceed in the order of crystallization process using methyl isobutyl ketone followed by crystallization process using normal-heptane. The crystallization step can be carried out by repeating the crystallization process one or more times, which proceeds in the order of 'adding crystallization solvent and then stirring'. In this manner, when the crystallization process is repeated while adding the solvent multiple times instead of adding the solvent at once, it is possible to obtain even the crystals that adhere to the wall surface during the solvent addition process, thereby preventing a decrease in yield, and effectively removing by-products remaining after the reaction. Additionally, it is possible to crystallize an uncrystallized product through repeated crystallization processes. Thereby, compared with a process of purifying in a slurry form using a solvent such as ethyl acetate, a high-purity compound can be obtained in a high yield.

The crystallization solvent used in one crystallization process may be used in an amount of volume(mL/g) of 3 to 20 times, more specifically, in an amount of volume(mL/g) of 3 to 15 times, relative to the weight of the compound represented by Chemical Formula 1-5. One crystallization process may be carried out by stirring at a temperature range of 5 to 30 °C for 10 minutes to 3 hours. The process time can be appropriately adjusted, for example, by increasing it when the number of repetitions of the crystallization process is small, and decreasing it when the number of repetitions is large.

That is, when all the steps of purifying and crystallizing a reaction product of the step 3 are carried out to prepare the compound represented by Chemical Formula 1-6, the step 3 may include the following steps, but is not limited to these steps:
a step of reacting a compound represented by Chemical Formula 1-5 with an acid;
a step of adding water and dichloromethane to discard the organic layer (lower layer) and separate and extract the aqueous layer (upper layer), a step of adding water and ethyl acetate to discard the aqueous layer (lower layer) and separate the organic layer (upper layer), and a step of vacuum concentrating and/or azeotropically concentrating the purified mixture; and
a step of crystallizing the concentrate.

After crystallizing and purifying the compound represented by Chemical Formula 1-6, the compound can be dried at 30 to 60 °C for 10 to 14 hours to reduce the moisture content contained in the compound represented by Chemical Formula 1-6. More specifically, if the moisture content of the compound represented by Chemical Formula 1-6 can be significantly reduced by drying at 50 °C or less, the conversion rate of the next step (i.e., step 4 below) can be increased.

### (Step 4)

The step 4 is an acrylation reaction step in which the compound represented by Chemical Formula 1-6 and the compound represented by Chemical Formula 1-7 are reacted in the presence of a base to prepare the compound represented by Chemical Formula 1.

In the step 4, the compound represented by Chemical Formula 1-7 can be used in an amount of 0.1 to 10 equivalents based on 1 equivalent of the compound represented by Chemical Formula 1-6. Specifically, the compound represented by Chemical Formula 1-7 can be used in an amount of 0.2 to 5 equivalents, 0.5 to 5 equivalents, 0.5 to 3 equivalents, 0.5 to 2 equivalents, 0.5 to 1.5 equivalents, 1 to 1.5 equivalents, 1 to 1.1 equivalents, or 1.1 equivalents based on 1 equivalent of the compound represented by Chemical Formula 1-6.

As the base used in the reaction of the step 4, at least one selected from the group consisting of potassium carbonate, sodium hydroxide, lithium hydroxide, potassium hydroxide, triethylamine, diisopropylamine, diisopropylethylamine, sodium hydrogen carbonate, potassium hydrogen carbonate, cesium carbonate, sodium carbonate, sodium methylate and potassium butyrate may be used. Among these bases, it is preferable to use potassium carbonate, sodium carbonate, sodium hydrogen carbonate, or potassium hydrogen carbonate in terms of the completion of reaction and the formation of by-products.

A mixed solvent of tetrahydrofuran(THF) and water can be used as the solvent for this reaction. At this time, the tetrahydrofuran may be used in an amount of volume (mL/g) of 5 to 40 times, or 5 to 10 times, relative to the weight of the compound represented by Chemical Formula 1-6, and the water may be used in an amount of volume (mL/g) of 2 to 10 times, or 2 to 5 times, relative to the weight of the compound represented by Chemical Formula 1-6.

In addition, the reaction of the step 4 may be carried out at a temperature of -10 °C to 50 °C for 1 to 5 hours. Preferably, the reaction may be carried out at a temperature of 0 °C or more, more preferably at a temperature of 0 °C or more and 10 °C or less. Thereby, it is preferable that all of the reaction reagents, such as the reactants and organic solvents used to suppress the increase in the reaction temperature during the reaction are cooled to a temperature of 0 °C or less and then used.

Meanwhile, after the reaction of the step 4 is completed, the method may further comprise a purification step of extracting the reaction product. Specifically, the reaction product may be extracted using ethyl acetate and water. That is, the compound represented by Chemical Formula 1 may be extracted using ethyl acetate and water after the reaction is completed. The extraction using ethyl acetate and water may be carried out by a method in which the mixture containing the reaction product and each solvent are stirred for 30 minutes or more, allowed to stand for 30 minutes or more and then separated into layers, so that an aqueous layer (lower layer), is discarded and an organic layer (upper layer) is used. In the case of such an extraction, impurities having a relative retention time of less than 1.0 separated by high-performance liquid chromatography (HPLC) can be effectively removed into an aqueous layer, compared with the case of extraction using a methylene chloride/water mixed solvent. Additionally, if necessary, a purification process may be carried out using activated carbon and/or silica-based materials to remove by-products or foreign substances.

In addition, the step 4 may further comprise a step of filtering the organic layer containing the extracted mixture and then concentrating it under vacuum. This vacuum concentration step is preferably carried out after the extraction step using ethyl acetate and/or water.

Preferably, the step 4 may further comprise a step of crystallizing a reaction product in order to purify the compound represented by Chemical Formula 1. This crystallization step is preferably carried out after the extraction purification and vacuum concentration steps. In other words, the step 4 may be carried out by adding a crystallization solvent to the concentrated product after the extraction purification step and stirring it at a temperature of about 20 to 40 °C for 30 minutes to 4 hours. Preferably, the crystallization step may be carried out at room temperature of 20 to 30 °C, or 20 to 25 °C. The process time can be appropriately adjusted by increasing it when the number of repetitions of the crystallization process is small, and decreasing it when the number of repetitions is large.

When a reaction product is prepared through crystallization using a crystallization solvent in this manner, unlike the case of purification using a silica column, it is possible to apply to industrialization, which is advantageous in terms of industrial mass production, and has the advantage of being able to obtain a high yield.

Specifically, the crystallization step can be carried out by crystallization using ethyl alcohol or normal-heptane as a crystallization solvent. If both of the solvents are used, the order is not limited. The crystallization step can be carried out by repeating the crystallization process one or more times, which proceeds in the order of 'adding crystallization solvent and then stirring'. In this manner, when the crystallization process is repeated while adding the solvent multiple times instead of adding the solvent at once, it is possible to obtain even the crystals that adhere to the wall surface during the solvent addition process, thereby preventing a decrease in yield, and effectively removing by-products remaining after the reaction. Additionally, it is possible to crystallize an uncrystallized product through repeated crystallization processes. Thereby, compared with a process of purifying in a slurry form using a solvent, a high-purity compound can be obtained in a high yield.

The crystallization solvent may be used in an amount of volume (mL/g) of 0.5 to 15 times, more specifically, in an amount of volume (ml/g) of 0.5 to 10 times, relative to the weight of the compound represented by Chemical Formula 1-6. One crystallization process may be carried out by stirring at a temperature range of 5 to 30 °C, preferably at room temperature (20 to 25 °C) for 10 minutes to 2 hours. The progress time may be appropriately adjusted, for example, by increasing it when the number of repetitions of the crystallization process is small, and decreasing it when the number of repetitions is large.

Meanwhile, in order to crystallize more quickly, the crystal of the compound represented by Chemical Formula 1, which is the reaction product, may be added as a seed together with the solvent one or more times during the crystallization process. The seed may be added in an amount of 0.01 parts by weight or less based on 1 part by weight of the compound represented by Chemical Formula 1-6.

That is, when all the steps of purifying and crystallizing a reaction product of the step 4 is carried out in sequence to prepare the compound represented by Chemical Formula 1, the step 4 may include the following steps, but is not limited to these steps:
a step of reacting a compound represented by Chemical Formula 1-6 and a compound represented by Chemical Formula 1-7 in the presence of a base;
a step of adding ethyl acetate and water, discarding an aqueous layer (lower layer) and separating and extracting an organic layer (upper layer), and a step of vacuum concentrating the purified mixture; and
a step of crystallizing the concentrate.

After the compound represented by Chemical Formula 1 is crystallized, it can be dried at 30 to 60 °C for 8 to 14 hours to reduce the moisture content contained in the compound represented by Chemical Formula 1. More specifically, it can be dried at 50 °C or less.

### [ADVANTAGEOUS EFFECTS]

As mentioned above, the preparation method according to the present disclosure has the advantages that it can prepare 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one in a high yield even through a shortened process, enables mass production, and improves the safety of the preparation process by using safer reactants than those used in conventional manufacturing methods.

### [DETAILED DESCRIPTION OF THE EMBODIMENTS]

Hereinafter, the present disclosure will be described in more detail with reference to Examples. However, the following Examples are for illustrative purposes only, and the scope of the present disclosure is not intended to be limited by the following Examples. In addition, in the following Examples, "volume" below can be understood to mean the ratio of the volume to the weight of the starting material in each step, "parts by weight" can be understood to mean the ratio of the weight relative to the starting material in each step, and "eq" can be understood to mean the equivalent of each material.

### Example 1: Preparation of Compound represented by Chemical Formula 1

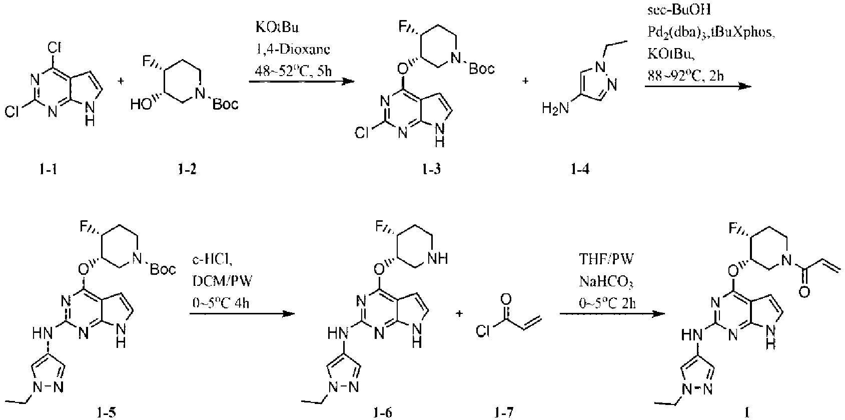

### Step 1) Preparation of Compound 1-3

2,4-Dichloro-7H-pyrrolo[2,3-d]pyrimidine (100.00 g, 1 eq) as Compound 1-1 and tert-butyl(3S,4R)-4-fluoro-3-hydroxypiperidine-1-carboxylate (122.45 g, 1.05 eq) as Compound 1-2 were dissolved in a mixture of 1000.0 mL(10 volume) of 1,4-dioxane and 10.0 mL(0.1 volume) of purified water, potassium tert-butoxide (179.05 g, 3 eq) was added thereto, and the mixture was stirred at 48 to 52 °C for 5 hours to complete the reaction.

Then, 1000.0 mL(10 volume) of ethyl acetate and 500.0 mL(5 volume) of purified water were added thereto, and the mixture was stirred for at least 30 minutes, allowed to stand for at least 30 minutes, and then separated into layers to discard an aqueous layer (lower layer). 500.0 mL(5 volume) of purified water was added to the remaining organic layer, and the mixture was stirred for at least 30 minutes, allowed to stand for at least 30 minutes and then separated into layers to discard an aqueous layer (lower layer). The remaining organic layer was vacuum concentrated at an external temperature of 50 °C or less, and 200.0 mL(2 volume) of 1,4-dioxane was added thereto, and the mixture was azeotropically concentrated at an external temperature of 50 °C or less.

200.0 mL(2 volume) of 1,4-dioxane was further added, and then the internal temperature was raised to 40~50 °C to completely dissolve the mixture, and 400.0 mL(4 volume) of purified water was added and stirred at room temperature for at least 1 hour. Additionally, 600.0 mL(6 volume) of purified water was added thereto and stirred for at least 1 hour. The resulting mixture was filtered under reduced pressure using a filter, and washed with 500.0 mL(5 volume) of purified water. This was vacuum dried at an external temperature of 50 °C for 12 hours to obtain tert-butyl (3S,4R)-3-((2-chloro-7H-pyrrolo[2,3-d]pyrimidin-4-yl)oxy)-4-fluoropiperidine-1-carboxylate (181.4 g, yield: 92.0%) as Compound 1-3.
1H NMR (500 MHz, CD₃OD): δ 7.206 (s, 1H), 6.552 (s, 1H), 5.461 (s, 1H), 5.093-4.997 (d, 1H), 3.908-3.793 (d, 2H), 3.559 (s, 2H), 2.210 (s, 1H), 1.926 (s, 1H), 1.287(s, 9H)

### Step 2) Preparation of Compound 1-5

Compound 1-3 (181.40 g, 1 eq) obtained from the step 1, 2-di-tert-butylphosphino-2',4',6'-triisopropylbiphenyl (8.31 g, 0.04 eq), tris(dibenzylideneacetone)dipalladium(0) (8.96 g, 0.02 eq) and potassium tert-butoxide (164.73 g, 3 eq) were added to 907.2 mL(5 volume) of sec-butyl alcohol, and then stirred at room temperature. Then, 1-ethyl-1H-pyrazol-4-amine (81.58 g, 1.5 eq) as Compound 1-4 was added, and then 181.4 mL(1 volume) of sec-butyl alcohol was added while washing the reactor walls and injection line so that all remaining reagents and raw materials would be used in the reaction. The resulting mixture was stirred at an internal temperature of 88 to 92 °C for 2 hours to complete the reaction.

Then, 907.2 mL(5 volume) of purified water and 907.2 mL(5 volume) of ethyl acetate were added thereto, and the mixture was filtered at an internal temperature of 50 °C, and washed with 907.2 mL(5 volume) of ethyl acetate. 907.2 mL(5 volume) of purified water was added once more to the filtrate, stirred for at least 30 minutes, allowed to stand for more than 30 minutes, and then separated into layers to discard an aqueous layer (lower layer). 907.2 mL(5 volume) of purified water was added to the remaining organic layer, stirred for at least 30 minutes, allowed to stand for at least 30 minutes, and then separated into layers to discard an aqueous layer (lower layer). 544.3 mL(3 volume) of 25 wt.% sodium chloride aqueous solution was added to the remaining organic layer, stirred for at least 30 minutes, allowed to stand for at least 30 minutes, and then separated into layers to discard an aqueous layer (lower layer). This was filtered and then washed with 907.2 mL(5 volume) of ethyl acetate. The filtrate was concentrated under vacuum at an external temperature of 60 °C or less, and then 544.3 mL(3 volume) of ethyl acetate was added. The mixture was then azeotropically concentrated at an external temperature of 60 °C or less to obtain tert-butyl (3S,4R)-3-((2-((1-ethyl-1H-pyrazol-4-yl)amino)-7H-pyrrolo[2,3-d]pyrimidin-4-yl)oxy)-4-fluoropiperidine-1-carboxylate (217.9 g, yield: 100.0%) as Compound 1-5. The obtained compound was used in the next step without purification.

### Step 3) Preparation of Compound 1-6

The concentrated residue of Compound 1-5 (217.9 g, 1 equivalents) obtained from the step 2 was added to and dissolved in 1090.0 mL(6 volume) of dichloromethane. The solution was cooled to 0 to 5 °C, and 414.2 mL(2.28 volume) of concentrated hydrochloric acid was slowly added so that the internal temperature did not exceed 5 °C, and then the mixture was stirred at 0 to 5 °C for 4 hours to complete the reaction.

Then, 1090.0 mL(6 volume) of dichloromethane and 1090.0 mL(6 volume) of purified water were added thereto, and the mixture was stirred for at least 30 minutes, allowed to stand for at least 30 minutes, and then separated into layers to discard an organic layer (lower layer). 1090.0 mL(6 volume) of dichloromethane was added to the remaining aqueous layer, and the mixture was stirred for 30 minutes or more, and allowed to stand for at least 30 minutes, and then separated into layers to discard an organic layer (lower layer). This was filtered, washed with 1090.0 mL(6 volume) of purified water, and 2179.9 mL(12 volume) of ethyl acetate was added and cooled to 0 to 5 °C. Then, about 1300.0 mL(7.17 volume) of 8N sodium hydroxide solution was added to adjust the pH to 9 to 11, allowed to stand for at least 30 minutes, and then separated into layers to discard an aqueous layer (lower layer). Additionally, 1090.0 mL(6 volume) of purified water was added, and the mixture was stirred for at least 30 minutes, allowed to stand for more than 30 minutes, and then separated into layers to discard an aqueous layer (lower layer). Vacuum concentration was then performed at an external temperature of 50 °C or less, 654.0 mL(3.6 volume) of methyl isobutyl ketone was added, and the mixture was azeotropically concentrated.

Additionally, 1090.0 mL(6 volume) of methyl isobutyl ketone was added, and the mixture was refluxed and stirred for at least 1 hour, and then the internal temperature was cooled to 20 to 25 °C, and 2179.9 mL(12 volume) of normal-heptane was slowly added and stirred for 2 hours. This was filtered and washed with 1090.0 mL(6 volume) of normal-heptane, and the washed filtrate was dried under vacuum at an external temperature of 50 °C or less for 12 hours to obtain 126.75 g (2 step in-situ yield: 75.0%) of N-(1-ethyl-1H-pyrazol-4-yl)-4-(((3S,4R)-4-fluoropiperidin-3-yl)oxy)-7H-pyrrolo[2,3-d]pyrimidin-2-amine as Compound 1-6.
1H NMR (500 MHz, CD₃OD): δ 8.902 (s, 1H), 7.906 (s, 1H), 7.528(s, 1H), 6.932 (s, 1H), 6.277 (s, 1H), 5.375-5.330 (d, 1H), 5.166-5.066 (d, 1H), 4.104-4.061 (d, 2H), 3.011-3.002 (d, 2H), 2.826-2.664 (dd, 2H), 2.006-1.776 (m, 2H), 1.370 (s, 3H)

### Step 4) Preparation of Compound 1

Compound 1-6 (126.75 g, 1 eq, 1 weight part) obtained from the step 3, and sodium hydrogen carbonate (61.66 g, 2 eq) were added to a mixed solution of 760.5 mL(6 volume) of tetrahydrofuran and 316.9 mL(2.5 volume) of purified water, and then cooled to 0 to 5 °C, and stirred for 1 hour. Acryloyl chloride (6.54 g, 1.1 eq) as Compound 1-7 was dissolved in 190.1 mL(1.5 volume) of tetrahydrofuran and slowly added so as not to exceed 5 °C. After the addition was completed, the mixture was stirred at 0 to 5 °C for 2 hours to complete the reaction.

Then, 1267.5 mL(10 volume) of purified water and 1267.5 mL(10 volume) of ethyl acetate were added thereto, and the mixture was stirred for at least 30 minutes, allowed to stand for at least 30 minutes, and then separated into layers to discard an aqueous layer (lower layer). This was filtered and washed with 380.3 mL(3 volume) of ethyl acetate. The filtrate was concentrated under vacuum at an external temperature of 50 °C or less, then 380.3 mL(3 volume) of ethyl alcohol was added thereto, and the mixture was concentrated under vacuum at an external temperature of 60 °C or less.

Additionally, 316.9 mL(2.5 volume) of ethyl alcohol was added, and then stirred at 20 to 25 °C to dissolve the mixture. 1.27 g (0.01 weight part) of Compound 1 as a seed was added at 20 to 25 °C, and stirred for 2 hours. When precipitation occurred sufficiently, 63.4 mL(0.5 volume) of ethyl alcohol was added, the fluidity was confirmed, followed by stirring for 2 hours. Then, 1140.7 mL(9 volume) of normal-heptane was added, and the mixture was stirred for 2 hours, and the crystallized liquid was filtered and then washed with 253.5 mL(2 volume) of normal-heptane. After washing, the filtrate was put in a dryer and vacuum-dried for 10 hours at an external temperature of 50 °C or less to obtain 114.34 g (yield: 78%) of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one as Compound 1.
1H NMR (500 MHz, CD₃OD): δ 7.678 (s, 1H), 7.543-7.491 (d, 1H), 6.612-6.424 (m, 2H), 6.320-5.523 (m, 3H), 5.360-5.241 (m, 1H), 5.185-5.007 (m, 1H), 4.709-3.400 (m, 6H), 2.192-1.839 (m, 2H), 1.358-1.303 (m, 3H)

### Comparative Example 1

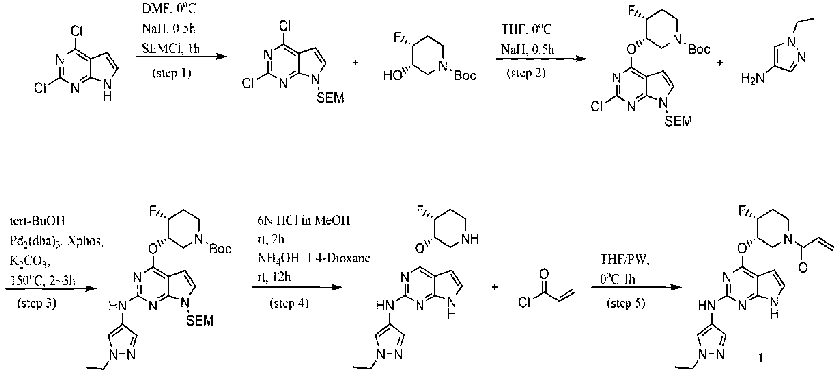

### Step 1) Preparation of 2,4-dichloro-7-((2-(trimethylsilyl)ethoxy)methyl)-7H-pyrrolo[2,3-d]pyrimidine

After 2,4-dichloro-7H-pyrrolo[2,3-d]pyrimidine (1.0 g, 5.3 mmol) was dissolved in N,N-dimethylformamide (10.0 mL), sodium hydride (234.0 mg, 5.9 mmol) was added thereto at 0 °C, and the mixture was stirred for 30 minutes. (2-(Chloromethoxy)ethyl)trimethylsilane (975.0 mg, 5.9 mmol) was added to the reaction mixture, and then stirred at room temperature for 1 hour. Ethyl acetate was added, and then distilled water was added to separate an organic layer. The organic layer was treated with sodium sulfate, filtered, and then concentrated under reduced pressure. The residue was separated by column chromatography to obtain 1.7 g (yield: 100.0%) of the title compound.

### Step 2) Preparation of tert-butyl (3S,4R)-3-((2-chloro-7-((2-(trimethylsilyl)ethoxy)methyl)-7H-pyrrolo[2,3-d]pyrimidin-4-yl)oxy)-4-fluoropiperidine-1-carboxylate

After tert-butyl (3S,4R)-4-fluoro-3-hydroxypiperidine-1-carboxylate (826.6 mg, 3.8 mmol) was dissolved in tetrahydrofuran (10.0 mL), sodium hydride (180.9 mg, 4.5 mmol) was added thereto at 0°C, and stirred for 30 minutes. 2,4-Dichloro-7-((2-(trimethylsilyl)ethoxy)methyl)-7H-pyrrolo[2,3-d]pyrimidine (1.2 g, 3.8 mmol) was added to the reaction mixture, and stirred at room temperature for 2 hours. Ethyl acetate was added, and then distilled water was added to separate an organic layer. The organic layer was treated with sodium sulfate, filtered, and then concentrated under reduced pressure. The residue was separated by column chromatography to obtain 1.6 g (yield: 83.1%) of the title compound.

### Step 3) Preparation of tert-butyl (3S,4R)-3-((2-((1-ethyl-1H-pyrazol-4-yl)amino)-7-((2-(trimethylsilyl)ethoxy)methyl)-7H-pyrrolo[2,3-d]pyrimidin-4-yl)oxy)-4-fluoropiperidine-1-carboxylate

Tert-butanol (40.0 mL) was added to tert-butyl (3S,4R)-3-((2-chloro-7-((2-(trimethylsilyl)ethoxy)methyl)-7H-pyrrolo[2,3-d]pyrimidin-4-yl)oxy)-4-fluoropiperidine-1-carboxylate (1.4 g, 2.7 mmol) and 1-ethyl-1H-pyrazol-4-amine (333.8 mg, 3.0 mmol). Tris(dibenzylideneacetone)dipalladium (125.0 mg, 0.1 mmol), 2-dicyclohexylphosphino-2',4',6'-triisopropylbiphenyl (128.6 mg, 0.3 mmol) and potassium carbonate (754.6 mg, 5.5 mmol) were added, and then stirred at 150 °C for 2~3 hours, and cooled to room temperature. Ethyl acetate was added and then distilled water was added to separate an organic layer. The organic layer was treated with sodium sulfate, filtered, and then concentrated under reduced pressure. The residue was separated by column chromatography to obtain 1.3 g (yield: 83.4%) of the title compound.

### Step 4) Preparation of N-(1-ethyl-1H-pyrazol-4-yl)-4-(((3S,4R)-4-fluoropiperidin-3-yl)oxy)-7H-pyrrolo[2,3-d]pyrimidin-2-amine

6N hydrochloric acid solution (10.0 mL) dissolved in methanol was added to tert-butyl (3S,4R)-3-((2-((1-ethyl-1H-pyrazol-4-yl)amino)-7-((2-(trimethylsilyl)ethoxy)methyl)-7H-pyrrolo[2,3-d]pyrimidin-4-yl)oxy)-4-fluoropiperidine-1-carboxylate (1.3 g, 2.3 mmol), and then the mixture was stirred at room temperature for 2 hours. The reaction product was concentrated, and then 1,4-dioxane (20.0 mL) and ammonia water (10.0 mL) were added to the residue. The resulting mixture was stirred at room temperature for 12 hours, and the reaction product was concentrated to obtain 785.8 mg (yield: 100.0%) of the title compound without further purification.

### Step 5) Preparation of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one

N-(1-Ethyl-1H-pyrazol-4-yl)-4-(((3S,4R)-4-fluoropiperidin-3-yl)oxy)-7H-pyrrolo[2,3-d]pyrimidin-2-amine (785.8 mg, 2.3 mmol) and sodium hydrogen carbonate (599.8 mg, 6.9 mmol) was dissolved in tetrahydrofuran/distilled water (15.0 mL / 3.0 mL), and then acryloyl chloride (212.7 uL, 2.6 mmol) was added at 0 °C. The reaction mixture was stirred at 0 °C for 1 hour. Ethyl acetate was added, and then distilled water was added to separate an organic layer. The organic layer was treated with sodium sulfate, filtered, and then concentrated under reduced pressure. The residue was separated by column chromatography to obtain 250.0 mg (yield: 27.5%) of the title compound as Compound 1.
1H NMR (500 MHz, CD₃OD) δ 7.98 (s, 1H), 7.57-7.55 (m, 1H), 6.88-6.45 (m, 2H), 6.30-5.98 (m, 2H), 5.80-5.44 (m, 2H), 5.20-5.05 (m, 1H), 4.40-4.12 (m, 3H), 4.05-3.52 (m, 3H), 2.24-2.21 (m, 1H), 2.01-1.94 (m, 1H), 1.47-1.43 (m, 3H)

### Experimental Example

The purity of the compound represented by Chemical Formula 1 prepared by the preparation method of Examples and Comparative Examples was measured as follows, and is shown in Table 2 below together with the yield.

### (1) Method for measuring purity (related substance)

When tested according to the following test method, the total amount of related substances in 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one was 1.0% or less. For unknown related substances detected at levels of 0.04% or more, the peak names were expressed as relative peak retention times, and the detected amount was recorded together.

### 1) Diluent

Acetonitrile · purified water mixture (weight ratio 4:1)

### 2) Preparation of standard solution (1 time)

About 30 mg of 1-((3S,4R)-3-((2-((1-ethyl-1H-pyrazol-4-yl)amino)-7H-pyrrolo[2,3-d]pyrimidin-4-yl)oxy)-4-fluoropiperidin-1-yl)prop-2-en-1-one standard was precisely weighted, and put in a 100 mL volumetric flask, and the diluent was added thereto, and dissolved completely and aligned with the marked line. A proper amount of the solution was taken, filtered, and the filtrate was used as a standard solution.

### 3) Preparation of test solution (3 times)

About 30 mg of this drug was precisely weighted, and put in a 100 mL volumetric flask, and the diluent was added thereto, and dissolved completely and aligned with the marked line. A proper amount of the solution was taken, filtered, and the filtrate was used as a standard solution.

This solution was prepared three times each and used as test solutions 1, 2, and 3.

### 4) Preparation of sensitivity confirmation solution (1 time)

4.0 mL of the test solution was precisely taken, and put in a 100 mL volumetric flask, and the diluent was added thereto, and dissolved completely and aligned with the marked line. 1.0 mL of this solution was precisely taken, and put in a 100 mL volumetric flask, and the diluent was added thereto, and dissolved completely and then aligned with the marked line. This solution was used as a sensitivity confirmation solution.

### 5) Operating conditions

10 µL of each of the blank solution, standard solution, and test solution was tested according to the liquid chromatography method of the General Test Methods of the Korean Pharmacopoeia under the following operating conditions.

### [Operating conditions]

- Detector: UV spectrophotometer (measurement wavelength: 235 nm)
- Column: X-Bridge C18 (150 x 4.6 mm, 3.5 µm) or equivalent column
- Flow rate: 1.0 mL/min
- Injection volume: 10 µL
- Column temperature: 40°C
- Mobile phase: Mobile phase A¹⁾ and mobile phase B²⁾ were controlled according to concentration gradient as shown in Table 1 below.

1) Mobile phase A: A solution in which 1.0 mL of trifluoroacetic acid was precisely weighted at room temperature (20°C), and added to and dissolved in 1000 mL of water
2) Mobile phase B: A solution in which 1.0 mL of trifluoroacetic acid was precisely weighted at room temperature (20°C), and added to and dissolved in 1000 mL of acetonitrile

**[Table 1]**

| Time (min) | Mobile phase A(%) | Mobile phase B(%) |
|---|---|---|
| 0.00 | 95 | 5 |
| 20.00 | 5 | 95 |
| 25.00 | 5 | 95 |
| 25.10 | 95 | 5 |
| 30.00 | 95 | 5 |

**[Table 2]**

| Example 1 | Yield(%) / Purity(%) | Comparative Example 1 | Yield(%) / Purity(%) |
|---|---|---|---|
| - | - | Step 1 | 100 / - |
| Step 1 | 92.0 / 92.6 | Step 2 | 83.1 / 89.3 |
| Step 2 | 100 / 80.8 | Step 3 | 83.4 / - |
| Step 3 | 75 / 98.7 | Step 4 | 100 / 95.7 |
| Step 4 | 78 / 99.3 | Step 5 | 27.5 / 95.9 |
| Final | 53.8 / 99.6 | Final | 19.06 / 95.9 |

As shown in Table 2, it is confirmed that when a compound represented by Chemical Formula 1 is prepared by the preparation method of Examples, unlike the preparation method of Comparative Example 1 in which the prepared compound are separated and purified through a column, the steps 2 and 3 are performed in-situ, and the compound are separated and purified through crystallization, which is advantageous for industrial production, and has a better yield than Comparative Example 1.

Therefore, it is confirmed that the preparation method of Examples according to the present disclosure makes it possible to industrially massproduce the compound represented by Chemical Formula 1 in high quality.

## Claims

1. A method for preparing a compound represented by the following Chemical Formula 1, the method comprising:
(step 1) reacting a compound represented by the following Chemical Formula 1-1 and a compound represented by the following Chemical Formula 1-2 in the presence of a base to prepare a compound represented by the following Chemical Formula 1-3;
(step 2) reacting a compound represented by the following Chemical Formula 1-3 and a compound represented by the following Chemical Formula 1-4 in the presence of a palladium catalyst and a base to prepare a compound represented by the following Chemical Formula 1-5;
(step 3) reacting a compound represented by the following Chemical Formula 1-5 with an acid to prepare a compound represented by the following Chemical Formula 1-6; and
(step 4) reacting a compound represented by the following Chemical Formula 1-6 and a compound represented by the following Chemical Formula 1-7 in the presence of a base to prepare a compound represented by the following Chemical Formula 1,
wherein at least one of the steps 1 to 4 further comprises a crystallization step: 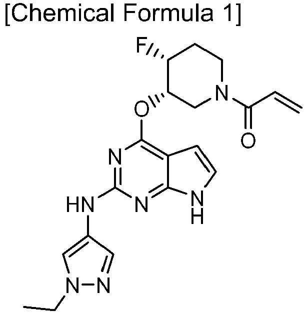 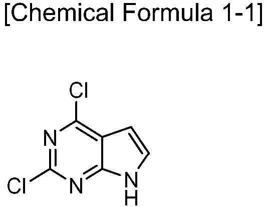 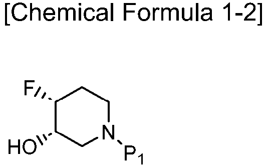 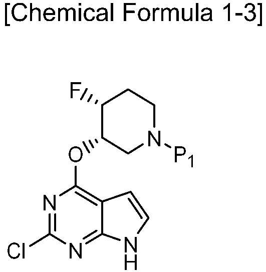 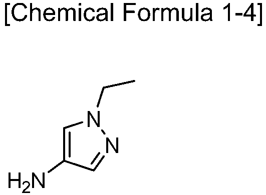 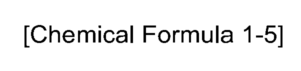 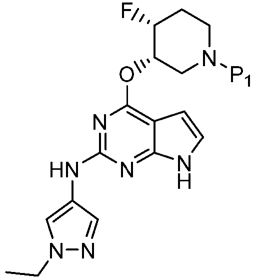 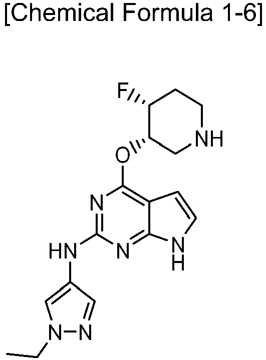 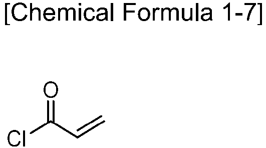 wherein in Chemical Formulas 1-1 to 1-7,
P₁ means a protecting group.

2. The preparation method according to claim 1,
wherein in the step 1, the compound represented by Chemical Formula 1-2 is used in an amount of 0.1 to 10 equivalents based on 1 equivalent of the compound represented by Chemical Formula 1-1.

3. The preparation method according to claim 1,
wherein in the step 1, the base is at least one selected from the group consisting of potassium tert-butoxide, sodium tert-butoxide, potassium methoxide, sodium methoxide, potassium tert-pentoxide, sodium tert-pentoxide, triethylamine, diisopropylethylamine, sodium hydroxide, potassium hydroxide, lithium hydroxide, potassium carbonate, calcium carbonate, sodium carbonate, potassium hydrogen carbonate, sodium hydrogen carbonate and cesium carbonate.

4. The preparation method according to claim 1,
wherein in the step 1, the base is used in an amount of 0.1 to 10 equivalents based on 1 equivalent of the compound represented by Chemical Formula 1-1.

5. The preparation method according to claim 1,
wherein in the step 1, the reaction is carried out in one or more organic solvents selected from the group consisting of tetrahydrofuran(THF), 1,4-dioxane, methanol, ethanol, isopropanol, 1-butanol, 2-butanol, acetone, methyl ethyl ketone, ethyl acetate, methyl tert-butyl ether, acetonitrile, toluene, heptane, 3-methyltetrahydrofuran, dichloromethane, methyl isobutyl ketone, dimethyl formamide(DMF), dimethyl acetamide(DMAC), and dimethyl sulfoxide(DMSO).

6. The preparation method according to claim 5,
wherein in the step 1, the reaction is carried out at 30 to 60 °C.

7. The preparation method according to claim 1,
wherein the step 1 further comprises a step of crystallizing a reaction product after reacting the compound represented by Chemical Formula 1-1 and the compound represented by Chemical Formula 1-2 in the presence of a base.

8. The preparation method according to claim 1,
wherein in the step 2, the compound represented by Chemical Formula 1-4 is used in an amount of 0.1 to 10 equivalents based on 1 equivalent of the compound represented by Chemical Formula 1-3.

9. The preparation method according to claim 1,
wherein in the step 2, the palladium catalyst is at least one selected from the group consisting of tris(dibenzylideneacetone)dipalladium(0), tetrakis(triphenylphosphine)palladium(0), bis[tris(2-methylphenyl)phosphine]palladium, palladium(II)acetate, 1,1-(bis(diphenylphosphino)ferrocene)palladium(II)dichloride, and palladium(II)chloride.

10. The preparation method according to claim 1,
wherein in the step 2, the base is at least one selected from the group consisting of potassium tert-butoxide, sodium tert-butoxide, potassium methoxide, sodium methoxide, potassium tert-pentoxide, sodium tert-pentoxide, triethylamine, diisopropylethylamine, sodium hydroxide, potassium hydroxide, lithium hydroxide, potassium carbonate, calcium carbonate, sodium carbonate, potassium hydrogen carbonate, sodium hydrogen carbonate and cesium carbonate.

11. The preparation method according to claim 1,
wherein in the step 2, the reaction is carried out in one or more organic solvents selected from the group consisting of tetrahydrofuran(THF), 1,4-dioxane, sec-butyl alcohol, methanol, ethanol, isopropanol, 1-butanol, 2-butanol, tert-butyl alcohol, acetone, methyl ethyl ketone, ethyl acetate, methyl tert-butyl ether, acetonitrile, toluene, xylene, heptane, 3-methyltetrahydrofuran, dichloromethane, methyl isobutyl ketone, dimethyl formamide(DMF), dimethyl acetamide(DMAC), and dimethyl sulfoxide(DMSO).

12. The preparation method according to claim 1,
wherein the reaction of the step 2 is carried out at 80 to 100 °C.

13. The preparation method according to claim 1,
wherein in the step 3, the acid is at least one selected from the group consisting of hydrochloric acid, acetic acid, trichloroacetic acid, trifluoroacetic acid, bromic acid, sulfuric acid, nitric acid and iodic acid.

14. The preparation method according to claim 1,
wherein in the step 3, the reaction is carried out in one or more organic solvents selected from the group consisting of ethyl acetate, dichloromethane, butyl acetate, trichloromethane, methanol, ethanol, isopropanol, 1-butanol, 2-butanol, acetone, methyl ethyl ketone, methyl tert-butyl ether, acetonitrile, toluene, heptane, tetrahydrofuran, 3-methyltetrahydrofuran, 1,4-dioxane, methyl isobutyl ketone, dimethylformamide(DMF), dimethylacetamide(DMAC), and dimethyl sulfoxide(DMSO).

15. The preparation method according to claim 1,
wherein the reaction of the step 3 is carried out at 0 °C to 50 °C.

16. The preparation method according to claim 1,
wherein the step 3 further comprises a step of crystallizing a reaction product after reacting the compound represented by Chemical Formula 1-5 with an acid.

17. The preparation method according to claim 1,
wherein in the step 4, the compound represented by Chemical Formula 1-7 is used in an amount of 0.1 to 10 equivalents based on 1 equivalent of the compound represented by Chemical Formula 1-6.

18. The preparation method according to claim 1,
wherein in the step 4, the base is at least one selected from the group consisting of potassium carbonate, sodium hydroxide, lithium hydroxide, potassium hydroxide, triethylamine, diisopropylamine, diisopropylethylamine, sodium hydrogen carbonate, potassium hydrogen carbonate, cesium carbonate, sodium carbonate, sodium methylate and potassium butyrate.

19. The preparation method according to claim 1,
wherein the reaction of the step 4 is carried out at -10 °C to 50 °C.

20. The preparation method according to claim 1,
wherein the step 4 further comprises a step of crystallizing a reaction product after reacting the compound represented by Chemical Formula 1-6 and the compound represented by Chemical Formula 1-7 in the presence of a base.

21. A method for preparing a compound represented by the following Chemical Formula 1,
comprising a compound represented by the following Chemical Formula 1-5 as an intermediate: 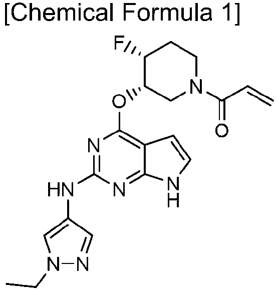 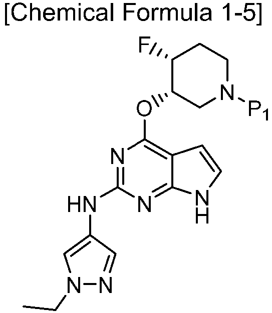 wherein in Chemical Formula 1-5,
P₁ means a protecting group.

22. The preparation method according to claim 21,
wherein P₁ is tert-butoxycarbonyl (Boc).

23. A compound represented by the following Chemical Formula 1-5: 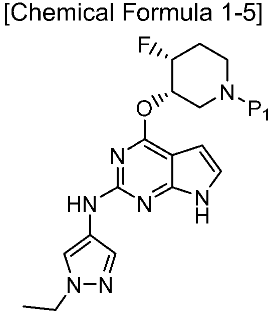 wherein in Chemical Formula 1-5,
P₁ means a protecting group.

24. The compound according to claim 23,
wherein P₁ is tert-butoxycarbonyl (Boc).
